# EUROPÄISCHE PATENTSCHRIFT

(11) **EP 3 451 211 B1**
(45) Veröffentlichungstag und Bekanntmachung des Hinweises auf die Patenterteilung: **28.02.2024**
(21) Anmeldenummer: 17188953.8
(22) Anmeldetag: 01.09.2017
(51) Int. Cl.: G16H 40/63, A61B 6/03, A61B 6/00, G16H 30/20, G16H 30/40

(54) **VERFAHREN UND STEUEREINRICHTUNG ZUR STEUERUNG EINES MEDIZINTECHNISCHEN BILDGEBENDEN SYSTEMS**
METHOD AND CONTROL DEVICE FOR CONTROLLING A MEDICAL IMAGING SYSTEM
PROCÉDÉ ET DISPOSITIF DE COMMANDE D'UN SYSTÈME TECHNIQUE D'IMAGERIE MÉDICALE

(43) Veröffentlichungstag der Anmeldung: 06.03.2019
(73) Patentinhaber: Siemens Healthineers AG, 91301 Forchheim (DE)
(72) Erfinder: Soza, Grzegorz, 90562 Heroldsberg (DE); Sühling, Michael, 91052 Erlangen (DE)
(74) Vertreter: Siemens Healthineers Patent Attorneys

(56) Entgegenhaltungen:
- WO-A2-2012/104786
- WO-A2-2013/179216
- US-A1- 2005 121 505
- US-A1- 2005 267 348
- SPANIER A B ET AL: "A new method for the automatic retrieval of medical cases based on the RadLex ontology", INTERNATIONAL JOURNAL OF COMPUTER ASSISTED RADIOLOGY AND SURGERY, SPRINGER, DE, Bd. 12, Nr. 3, 1. November 2016 (2016-11-01), Seiten 471-484, XP036159446, ISSN: 1861-6410, DOI: 10.1007/S11548-016-1496-Y [gefunden am 2016-11-01]

## Beschreibung

Die Erfindung betrifft ein Verfahren und eine Steuereinrichtung zur Steuerung eines medizintechnischen bildgebenden Systems, sowie ein solchermaßen gesteuertes medizintechnisches bildgebendes System.

Die optimale Erfassung und Rekonstruktion von CT-Scans ist ein grundlegender Schritt, um ein qualitativ hochwertiges Diagnoseergebnis zu erreichen.

Das Dokument WO 2013179216 A2 beschreibt die Bereitstellung eines Bildgebungsprotokolls basierend auf einer kodierten Beschreibung von Bildgebungsprotokollen und von klinischen Informationen die in ein Datenverarbeitungsformat überführt wurden.

In der derzeitigen klinischen Arbeit werden sowohl die Auswahl von Scanprotokollen und Kontrastprotokollen als auch die Regeln zur Bildrekonstruktion von diesen auf der Basis manuell definierter Konfigurationsregeln vorgenommen. Diese manuell definierten Konfigurationsregeln hängen von den Informationen aus radiologischen Informationssystemen ab oder werden von einem Techniker im Untersuchungsraum vollständig manuell für jeden einzelnen Patienten im Moment der Bildaufnahme durchgeführt. Informationen über aktuelle und andere Patienten, eine klinische Historie oder klinische Berichte werden zurzeit überhaupt nicht genutzt. Somit ist der Scan- und Rekonstruktionsprozess für jeden Patienten mit derselben klinischen Indikation jeweils der gleiche. Zumindest sofern an dem betreffenden medizintechnischen Gerät keine manuellen Einstellungen vorgenommen werden.

Dies ist nachteilhaft, da ein solches Vorgehen sehr zeitraubend ist und eine individuelle Einstellung für jeden zu untersuchenden Patienten kaum praktikabel ist.

Es ist eine Aufgabe der vorliegenden Erfindung, ein verbessertes Verfahren und eine entsprechend verbesserte Steuereinrichtung zur Verfügung zu stellen, mit dem die oben beschriebenen Nachteile vermieden werden.

Diese Aufgabe wird durch ein Verfahren gemäß Patentanspruch 1 sowie durch eine Steuereinrichtung gemäß Patentanspruch 12 und ein medizintechnisches bildgebendes System gemäß Patentanspruch 13 gelöst.

Das erfindungsgemäße Verfahren zur Steuerung eines medizintechnischen bildgebenden Systems, insbesondere zur automatischen Ermittlung von Steuerbefehlen für ein solches System, umfasst die folgenden Schritte.
- Bereitstellung eines Datensatzes eines Patienten.

Dieser Datensatz, der auch als "Patientendatensatz" bezeichnet werden kann, umfasst dabei Indikationsdaten. Diese Indikationsdaten sind Daten über eine medizinische Indikation, welche diesen Patienten betreffen, bzw. ein Krankheitsbild, welches ein Befunder bei diesem Patienten für wahrscheinlich erachtet. Die Indikationsdaten können auch bereits eine vorzunehmende Prozedur beinhalten, z.B. von einem Radiologieinformationssystem ("RIS") oder einer sogenannten Modality Worklist, in der RIS Terminologie auf DICOM Attribute abgebildet (DICOM = Digital Imaging and Communications in Medicine = Digitale Bildverarbeitung und Kommunikation in der Medizin) .
- Abbildung der Indikationsdaten in eine Ontologie.

Eine solche Abbildung wird auch als "Mapping" bezeichnet, und betrifft erfindungsgemäß zumindest Indikationsdaten, wobei je nach Anwendungsfall auch andere Daten in die Ontologie abgebildet werden können. Die betreffende Ontologie ist eine Ontologie im Sinne der Informatik. Sie kann auch als "Indikations-Ontologie" bezeichnet werden.

Ontologien in der Informatik, also im Sinne der Erfindung, sind sprachlich gefasste und formal geordnete Begriffe oder numerische Bezeichnungen zu medizinischen Daten und der zwischen ihnen bestehenden Beziehungen in im medizinischen Gegenstandsbereich. Sie werden dazu genutzt, textliche Informationen in digitalisierter und formaler Form einer Rechenvorrichtung zugänglich zu machen. Ontologien enthalten Inferenz- und Integritätsregeln, also Regeln zu Schlussfolgerungen und zur Gewährleistung ihrer Gültigkeit. Eine Ontologie stellt damit ein Netzwerk von Informationen mit logischen Relationen dar. Man könnte diesbezüglich auch von einer expliziten formalen Spezifikation einer Konzeptualisierung (Begriffsbildung) sprechen. Ontologien verfügen über eine hohe semantische Ausdrucksstärke, wodurch sie auch komplexe Datenmodelle oder Wissensrepräsentationen darstellen können.

Die Ontologie hat somit eine vordefinierte Struktur und kann zu den Indikationsdaten auch allgemeines, standardisiertes Wissen enthalten, wie z.B. RadLex. Die RadLex-Ontologie wurde von der "Radiological Society of North America" (RSNA) einer Vereinigung von Radiologen, medizinischen Physikern und weiteren medizinischen Wissenschaftlern aus 136 Staaten festgelegt und stellt einen oft verwendeten Standard dar. Ziel der Verwendung einer solchen Ontologie ist, textbasiertes Vorwissen über den Patienten, was zumindest die textlich gestalteten Indikationsdaten umfasst, auf eine standardisierte Wissensrepräsentation abzubilden ("zu mappen"). Für den gegebenen Patienten wird insbesondere eine Instanz dieser Ontologie mit patientenspezifischen Werten erzeugt.

Mit der Abbildung der Indikationsdaten in die Ontologie werden die Indikationsdaten direkt einer Rechenvorrichtung zugänglich gemacht, so dass diese die Indikationsdaten im Rahmen einer semantischen Logik bearbeiten und nutzen kann.

Die Abbildung der Indikationsdaten in die Ontologie kann beispielsweise durch semantische Analyse erfolgen, insbesondere umfassend den Abgleich von Textbestandteilen. Die Abbildung kann aber auch durch Konvertieren der Befunddaten vorgenommen werden oder durch direkte Interpretation der Befunddaten für eine weitere Verarbeitung mittels eines Interpreters erfolgen. Bevorzugt ist in diesem Rahmen der RadLex-Standard, bzw. RadLex Playbook. Dies ist eine Erweiterung der Radlex Ontologie und enthält ein Standardlexikon für radiologische Begrifflichkeiten und Bezeichnungen für Verfahrensschritte. Eine Möglichkeit ist, die "Verdachtsdiagnose" ("admitting diagnosis"), also diejenige Diagnose, die ausschlaggebend dafür ist, weshalb eine CT Untersuchung angefordert wird. Dies ist in der Regel ein von einem Arzt verfasster Freitext, der zunächst auf eine Ontologie abgebildet werden muss, um von einem Computersystem "verstanden" zu werden. Die Verdachtsdiagnose kann beispielsweise "Verdacht auf Herzinfarkt" lauten.

Es können ggf. ICD-10-Codes zur Beschreibung der Vorläufigen Diagnose verwendet werden, welche eine formale Definition des klinischen Zusammenhangs bzw. Kontexts enthalten, wobei eine Vorläufige Diagnose beispielsweise eine ICD-10-CM Diagnose gemäß Code J44.1 sein könnte, wie z.B. "Chronic obstructive pulmonary disease (COPD) with (acute) exacerbation".

Medizinische Berichte in Krankenhausinformationssystemen bzw. Radiologieinformationssystemen ("HIS" bzw. "RIS") und seit kurzem auch in syngo.via basieren oftmals auf dem HL7 CDA Standard, welcher klar definierte Technologien verwendet, wie z.B. SNOMED CT oder RadLex. Dadurch liegen bestehende Informationen und Ergebnisse über den Patienten in diesem Fall bereits in einem semantisch zugänglichen Format vor. Informationen können automatisch mit einer Patienten-ID des vorliegenden Scans aus einem Quellsystem (HIS, RIS, syngo.via) erhalten werden. In einem solchen Fall stellt die Abbildung der Indikationsdaten in die Ontologie eine Überprüfung dar, ob all diese Daten auch in einem korrekten Format für die Indikations-Ontologie vorliegen.
- Bereitstellung einer Steuerdaten-Bibliothek.

Diese Steuerdaten-Bibliothek umfasst Steuerdatensätze, die bevorzugt in einem symbolischen und/oder subsymbolischen Format vorliegen, also systemverständliche Begriffe bzw. numerische Werte enthalten. Solche Steuerdatensätze können Steuerprotokolle (Routinen zur Steuerung eines medizintechnischen bildgebenden Systems), Kontrastprotokolle (Routinen zur Steuerung der Verabreichung eines Kontrastmittels) oder auch Rekonstruktionsprotokolle (Routinen zur Nachbearbeitung der aufgenommenen Bilddaten) für zumindest ein medizintechnisches bildgebendes System sein. Es ist dabei möglich, dass die Steuerdaten-Bibliothek auch Steuerdatensätze für mehrere medizintechnische bildgebende Systeme umfasst. Die Steuerdaten-Bibliothek hat bevorzugt die Form einer Ontologie oder ist eine Ontologie, in der Steuerdatensätze abgebildet sind. Dies wird im weiteren Verlauf noch genauer beschrieben.

Die Steuerdaten-Bibliothek enthält also proprietäres Wissen, das spezifisch für die Steuerung zumindest eines medizintechnischen bildgebenden Systems ist. Diese wird in einem folgenden Schritt mit der Indikations-Ontologie verknüpft.
- automatische Ermittlung eines Steuerdatensatzes.

Dieser Steuerdatensatz wird dabei aus der Steuerdaten-Bibliothek in Abhängigkeit von den in die Ontologie abgebildeten Indikationsdaten ermittelt. Diese Ermittlung geschieht mittels einer Inferenz (logische Schlussfolgerungen) und/oder numerischen Modellierung. Bezüglich dieser Erfindung wird selbstverständlich von einer Inferenz im Sinne einer informatischen Semantik ausgegangen. Im Zusammenhang mit Ontologien sind die technischen Funktionsmechanismen einer Inferenz bekannt. Bei einer Inferenz werden Terme der Ontologie (Indikations-Ontologie) und der Steuerdaten-Bibliothek zueinander in Beziehungen gesetzt und logische Schlussfolgerungen abgeleitet, um den geeigneten Steuerdatensatz zu ermitteln. Bei der numerischen Modellierung geschieht die Ermittlung auf numerischem Wege. Numerische Modelle können z.B. Ergebnisse eines Verfahrens des Maschinellen Lernens oder statistischer Analysen sein. So können z.B. aus einer großen Menge an Indikationsdaten und jeweils zugehörigen, aus der klinischen Routine bestimmten Scan Protokollen, statistische oder "Machine Learning"- basierte Klassifikations-Modelle abgeleitet werden. Diese Modelle geben für gegebene Indikationsdaten numerische Wahrscheinlichkeiten für Steuerdatensätze aus. Würde zudem aus den semantischen Indikationsdaten eine "Kontrastmittel Allergie" identifiziert, würde über logische Schlussfolgerungen auf Grund von konzeptuellem Ontologie Wissen in der Steuerdaten-Bibliothek eine elektive oder notfallmäßige Prämedikation als Teil des Protokolls empfohlen werden.
- Steuerung des medizintechnischen bildgebenden Systems.

Das medizintechnische bildgebende System wird dabei mit dem ermittelten Steuerdatensatz gesteuert. Dabei werden mittels des Systems auf Basis der Steuerdaten in dem Steuerdatensatz in der Regel Bilddaten aufgenommen, die weiterverarbeitet oder direkt begutachtet werden können.

Im Folgenden wird der Verfahrensablauf anhand eines Beispiels verdeutlicht:
Bei einer Untersuchung eines Patienten stellt ein Arzt die Diagnose "Verdacht auf Herzinfarkt". Dieser in das System eingegebene Text wird im Rahmen der Erfindung in eine Ontologie abgebildet. Dies kann in einem sehr einfachen Fall dadurch geschehen, dass die einzelnen Worte des Textes analysiert und mit in dieser Ontologie festgelegten Begriffen verglichen werden. In dieser Ontologie könnte dann der Begriff "Herzinfarkt" mit einer Indikation verknüpft werden ("INDIKATION: HERZINFARKT", wobei die Zeichenkette "HERZINFARKT" eine dem System bekannte Zeichenkette ist). In RadLex Playbook wäre mit der Indikation "HERZINFARKT" ein bestimmter Steuersatz für ein CT Verknüpft, wobei die spezifischen Eigenschaften des in der betreffenden medizinischen Einrichtung benutzten CT-Systems im Steuerdatensatz berücksichtigt würden. Die Inferenz wäre dann, dass aufgrund der Indikation "HERZINFARKT" ein mit dieser Zeichenkette verknüpfter Steuerdatensatz für das verwendete CT-System ausgewählt würde. Dieser Steuerdatensatz könnte noch aufgrund zusätzlicher numerischer Daten verändert werden, oder die Wahl des Steuerdatensatzes könnte durch numerische Daten beeinflusst werden. Zum Beispiel könnte die Dosis zur Messung auf das Alter des Patienten angepasst werden (z.B. Kinder-Protokoll), wobei das Alter als numerischer Wert vorliegt. Es können also sowohl semantische Begriffe ausgewertet werden, als auch numerische Werte über eine Funktion in die Auswahl oder Konfiguration des Steuerdatensatzes einfließen.

Eine erfindungsgemäße Steuereinrichtung zur Steuerung eines medizintechnischen bildgebenden Systems, insbesondere nach dem erfindungsgemäßen Verfahren umfasst die folgenden Komponenten.

Eine Schnittstelle zur Bereitstellung eines Datensatzes eines Patenten. Wie oben bereits gesagt wurde, umfasst dieser Patientendatensatz die benötigten Indikationsdaten.

Eine Schnittstelle und/oder eine Datenbank zur Bereitstellung einer Steuerdaten-Bibliothek umfassend Steuerdatensätze für zumindest ein medizintechnisches bildgebendes System. Die Steuerdatenbibliothek kann dabei direkt in einem Datenspeicher der Steuervorrichtung vorliegen, also in besagter Datenbank, oder in einer externen Datenbank, wofür die besagte Schnittstelle zum Zugriff auf diese Datenbank benötigt wird.

Eine Überführungseinheit, die zur Überführung zumindest der Indikationsdaten in eine (Indikations-)Ontologie ausgelegt ist.

Eine Ermittlungseinheit, welche zur Ermittlung eines Steuerdatensatzes aus der Steuerdaten-Bibliothek in Abhängigkeit von den in die Ontologie abgebildeten Indikationsdaten ausgelegt ist. Die Ermittlungseinheit ist dabei dazu ausgebildet, die Ermittlung mittels einer semantischen Inferenz und/oder einer numerischen Modellierung durchzuführen.

Das erfindungsgemäße medizintechnische bildgebende System umfasst eine erfindungsgemäße Steuereinrichtung.

Ein Großteil der zuvor genannten Komponenten des Systems können ganz oder teilweise in Form von Softwaremodulen in einem Prozessor einer entsprechenden Steuereinrichtung realisiert werden. Eine weitgehend softwaremäßige Realisierung hat den Vorteil, dass auch schon bisher verwendete Infrastruktur auf einfache Weise durch ein Software-Update nachgerüstet werden kann, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung eines medizintechnischen bildgebenden Systems ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation bzw. zusätzliche Komponenten auch Hardware-Komponenten, wie z.B. Hardware-Schlüssel (Dongles etc.) zur Nutzung der Software, umfassen.

Bevorzugt ist auch ein Computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Rechnereinheit ausgeführt werden.

Weitere, besonders vorteilhafte Ausgestaltungen und Weiterbildungen der Erfindung ergeben sich aus den abhängigen Ansprüchen sowie der nachfolgenden Beschreibung. Dabei können die Merkmale zu Ausführungsformen einer Kategorie auch zur Charakterisierung von Ausführungsformen einer anderen Kategorie dienen. Beispielsweise kann die erfindungsgemäße Steuereinrichtung auch analog zu den abhängigen Verfahrensansprüchen oder Beschreibungsteilen weitergebildet sein, wobei Entsprechendes umgekehrt auch für die Verfahrensansprüche gilt. Es können insbesondere auch einzelne Merkmale verschiedener Ausführungsbeispiele bzw. Varianten zu neuen Ausführungsbeispielen bzw. Varianten kombiniert werden.

Bevorzugt umfasst die Steuerdaten-Bibliothek Steuerdatensätze für zumindest zwei bildgebende medizintechnische Systeme. Dies hat den Vorteil, dass für eine medizinische Einrichtung die Steuerdatensätze für mehrere bzw. alle medizintechnischen bildgebenden Geräte einer medizinischen Einrichtung in einer einzigen Steuerdaten-Bibliothek abgespeichert sein können. Vor der automatischen Ermittlung eines Steuerdatensatzes sollte vorteilhafterweise dann jedoch eine manuelle oder, ggf. auch teilweise, automatische Auswahl eines medizintechnischen bildgebenden Systems erfolgen, mit dem eine Aufnahme von Bereichen des Körpers des Patienten durchgeführt werden soll. Die Steuereinrichtung umfasst in diesem Fall bevorzugt eine Eingabeeinheit zur Auswahl eines medizintechnischen bildgebenden Systems, mit dem eine Aufnahme durchgeführt werden soll.

Somit kann vor, während oder nach der Bereitstellung der Indikationsdaten eine Auswahl des geeigneten medizintechnischen bildgebenden Systems vorgenommen werden und die nachfolgende Steuerung für das ausgewählte medizintechnische bildgebende System mit dem für dieses System ermittelten Steuerdatensatz erfolgen.

Bevorzugt werden zusätzlich zu den Indikationsdaten weitere Daten des Patienten auf die (Indikations-)Ontologie abgebildet. Bevorzugt sind dabei eine oder mehrere der folgenden Daten:
a) Weitere Daten im Datensatz des Patienten, insbesondere Daten der Gruppe Alter, Gewicht, Größe, ethnische Zugehörigkeit, Geschlecht.
b) Weitere Daten einer Patientengruppe, z.B. durchschnittliche Körpermaße, typisch Krankheitsbilder, medizinische Bilddaten.

Bevorzugt umfasst die (Indikations-)Ontologie zusätzliche medizinische Daten der Gruppe: Daten eines lexikalischen Werks, welche insbesondere aus einem Krankenhausinformationssystem und/oder einem Radiologieinformationssystem ("HIS" bzw. "RIS") abgerufen werden können, medizinische Vorgeschichte, Vorerkrankungen, allgemeiner Gesundheitszustand, z.B. von HIS/RIS, syngo.via, Kontrastprotokolle, Informationen über verabreichte Kontrastmittel oder Scanprotokolle, z.B. CT- oder MRT-Protokolle früherer Scans und ggf. auch Rekonstruktionen. Die Scanprotokolle können beispielsweise in Form eines DICOM-Headers eines Bildes oder einer unabhängigen DICOM Datei vorliegen.

Bevorzugt umfasst die Steuerdaten-Bibliothek Daten im RadLex Playbook-Standard und würde damit, wie bereits oben gesagt wurde, eine Ontologie darstellen oder umfassen. Diese Daten basieren bevorzugt auf einem in der Vergangenheit erstellten semantischen Scanprotokoll und/oder aktuellen oder vorangegangenen Untersuchungen des Patienten oder einer Patientengruppe.

RadLex Playbook umfasst semantisch definierte (CT-)Scanprotokolle. Diese Scanprotokolle beinhalten standardisierte, direkt zugängliche semantische Informationen über bildgebende Untersuchungen. In dem Fall, dass solche Informationen nur als Text erhältlich sind, werden bevorzugt Referenz-Ontologien wie z.B. RadLex or SNOMED CT zum Aufgliedern solcher Texte in semantische, also computerverständliche, Informationen verwendet.

Bevorzugt wird zur Ermittlung des Steuerdatensatzes eine modellierende Workflow-Ontologie, insbesondere im OWL-Standard, verwendet. Diese die Workflow-Ontologie modelliert dabei Schritte der Scanprozedur, benötigte Eingangs- und Ausgangsinformationen und verfügbare Steuerdaten für die Steuerdatensätze, wie z.B. Scanprotokolle, welche die gesamte Scan-Knowledge beinhalten. Die Workflow-Ontologie wird dabei ständig mit Informationen über medizinische Erfordernisse oder neue Steuerdaten für die Steuerdatensätze erweitert oder aktualisiert.

Die "Scan-Knowledge" umfasst das spezifische Wissen über ein bestimmtes Gerät, z.B. welches Protokoll mit welcher Parametrisierung für welche Untersuchung verwendet wird, welche Energie in welchem Fall zur Bildgebung eingestellt wird oder welche sonstigen Einstellungen am medizintechnischen System vorgenommen werden. In der Workflow-Ontologie ist also in der Regel das Wissen über die Steuerung eines CT-Systems oder einer Gruppe von CT-Systemen enthalten und eine Reihe von Steuerdatensätzen für verschiedene bildgebende Messungen.

Die Inferenz bzw. die numerische Modellierung nutzt dabei aus, dass die logischen Zusammenhänge der Daten des Patientendatensatzes und der Steuerdaten-Bibliothek zu den Indikationsdaten passen müssen und nur diejenigen Daten zur Ermittlung hinzugezogen werden, welche zu den Indikationsdaten passen. Der Begriff "passen" bezieht sich dabei auf eine Festlegung innerhalb der Ontologie, also eine voreingestellte Zuordnung von Begriffen bzw. semantischen Objekten. Beispielsweise werden Untersuchungen zu einem gebrochenen Bein bei einer med. Indikation auf eine Erkrankung des Herzens nicht berücksichtigt.

Alternativ oder ergänzend dazu nutzt die Inferenz bzw. die numerische Modellierung numerische Informationen des Patienten oder der Patientengruppe aus, um optimale Parameter für den Patienten zu erhalten, bevorzugt mittels maschineller Lernprozeduren wie z.B. "machine learning" oder "deep learning"). Beispielsweise wird aufgrund des Gewichts, der Größe oder des Alters des Patienten, wobei diese Daten als numerische Werte vorliegen, die Auswahl des Steuerdatensatzes beeinflusst oder der Steuerdatensatz angepasst. Dies kann beispielsweise mit einfachen mathematischen Funktionen geschehen.

Erfindungsgemäß wird nach der Steuerung des ausgewählten medizintechnischen bildgebenden Systems und einer Aufnahme eines Bilddatensatzes durch das medizintechnische bildgebende System der Bilddatensatz automatisch oder halbautomatisch rekonstruiert und/oder grafisch nachbearbeitet, basierend auf dem ermittelten Steuerdatensatz. Dazu wird bevorzugt zur Bildrekonstruktion eine modellierende Workflow-Ontologie verwendet, welche besonders bevorzugt ständig mit Informationen über Rekonstruktionsprotokolle erweitert oder aktualisiert wird. Bevorzugt ist die Bildrekonstruktion Teil der weiter oben beschriebenen Workflow Ontologie, die zur Auswahl des Steuerdatensatzes verwendet wird. Diese ist dazu vorzugsweise modular aufgebaut. Die Bildrekonstruktion kann auch bereits direkt Teil eines Steuerdatensatzes sein.

Um eine optimale Auswertung von Bilddatensätzen, die zur Untersuchung eines Patienten aufgenommen wurden, seitens eines Befunders zu ermöglichen, so dass dieser einfach in der Lage ist, eine verlässliche Diagnose zu stellen, ist bevorzugt, dass die vom medizintechnischen bildgebenden System aufgenommenen Bilder entsprechend der Indikationsdaten dem Befunder zur Verfügung stehen. Dazu müssen die Bilder in Abhängigkeit von den Indikationsdaten eine, ggf. jeweils unterschiedliche, spezielle Rekonstruktion erfahren und Zusammenstellung erhalten.

Gemäß einer bevorzugten Weiterbildung des Verfahrens wird nach der Steuerung des ausgewählten medizintechnischen bildgebenden Systems und einer Aufnahme eines Bilddatensatzes durch das medizintechnische bildgebende System dieser Bilddatensatz durch einen Messdatendarstellungs-Algorithmus automatisch für die Anzeige an einen Befunder aufbereitet. Dieser Messdatendarstellungs-Algorithmus kann basierend auf den Indikationsdaten und/oder dem Steuerdatensatz automatisch aus einer Darstellungsbibliothek ermittelt werden. Eine bevorzugte Steuereinrichtung umfasst dazu eine Datenbank mit dieser Darstellungsbibliothek bzw. eine Schnittstelle zum Zugriff auf eine solche Datenbank.

Bevorzugt werden dem Steuerdatensatz zusätzlich während oder nach seiner Ermittlung weitere Daten zugeordnet, , insbesondere basierend auf der (Indikations-)Ontologie enthaltend Daten des Patienten, einer Patientengruppe und/oder eines lexikalischen Werks, wobei diese weiteren Daten dem Messdatendarstellungs-Algorithmus Informationen zur Darstellung der Messdaten liefern.

Bevorzugt bereitet der Messdatendarstellungs-Algorithmus zusätzlich zu dem Bilddatensatz auch Daten des Datensatzes des Patienten, also nicht nur die Indikationsdaten, sondern weitere Daten zu dem Patienten, und insbesondere ebenfalls Daten einer lexikalischen Datenbank automatisch für die Anzeige an einen Befunder auf.

Ein Vorteil der Erfindung ist, dass eine automatische fallspezifische, Bildaufnahme und -rekonstruktion ermöglicht wird, in die insbesondere die klinische Historie aufgenommen werden oder in dieser berücksichtigt werden kann.

Auf diese Weise können einem Radiologen, der mit der Untersuchung befasst ist, automatisch optimale Bilder zur Verfügung gestellt werden.

Die Erfindung ist insbesondere für sogenannte Automatisierungs- und PACS-fähige Szenarien anwendbar, die das Ziel haben, fehlersichere Bildaufnahmen und Auswertungen zu ermöglichen.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 ein Blockdiagramm zur Verdeutlichung eines möglichen Ablaufs eines erfindungsgemäßen Verfahrens,
Figur 2 eine schematische Darstellung einer bevorzugten Steuereinrichtung,
Figur 3 ein Blockdiagramm zur Verdeutlichung des Ablaufs einer bevorzugten Ausführungsform des erfindungsgemäßen Verfahrens.

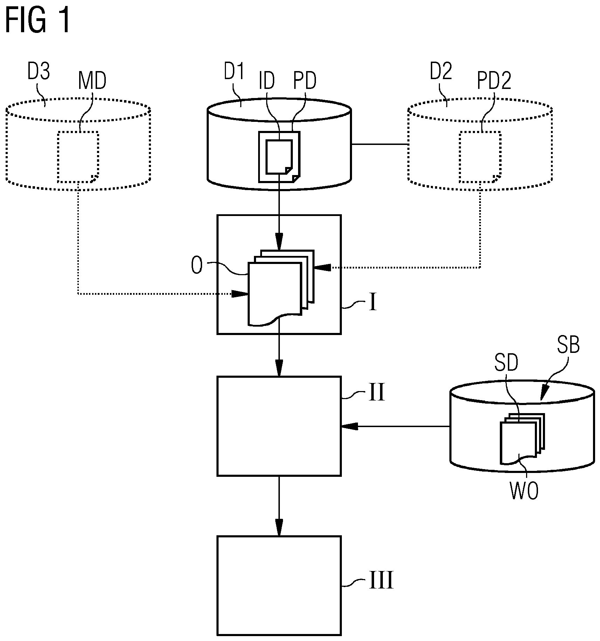

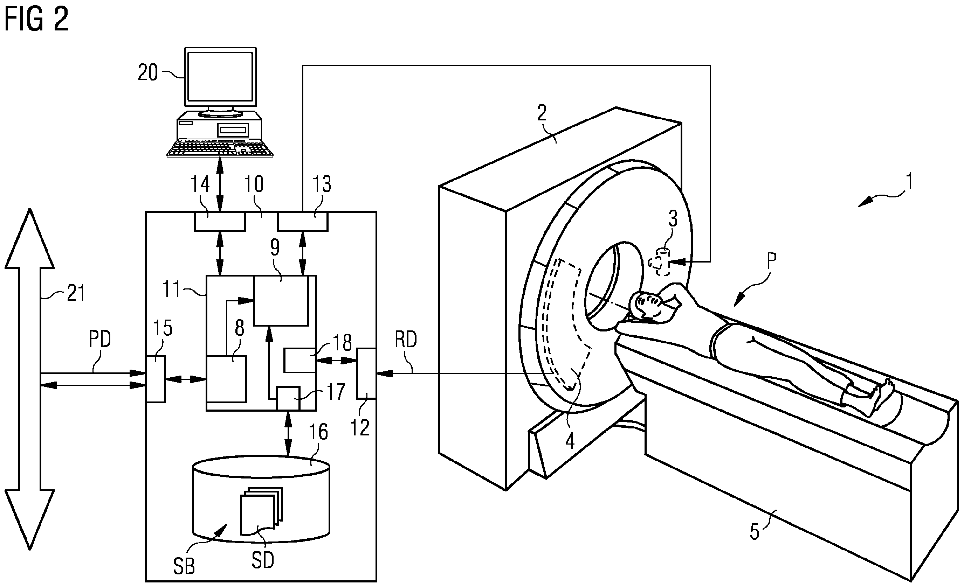

Figur 1 zeigt ein Blockdiagramm zur Verdeutlichung eines möglichen Ablaufs eines erfindungsgemäßen Verfahrens zur Steuerung eines medizintechnischen bildgebenden Systems 1, wie es z.B. in Figur 2 dargestellt ist.

Zunächst erfolgt eine Bereitstellung eines Patientendatensatzes PD enthaltend Indikationsdaten ID durch eine Datenbank D1. In diesem Falle werden in Schritt I die Indikationsdaten ID in eine (Indikations-)Ontologie O abgebildet. Je nach Anwendung könnten auch die Patientendaten PD ergänzend in die Ontologie O abgebildet werden.

Gestrichelt sind zwei weitere Datenbanken D2, D3 angedeutet, deren Daten optional ebenfalls in die Ontologie O abgebildet werden könnten. Die rechte Datenbank D2 enthält weitere Patientendaten PD2, z.B. Befunddaten eines anderen Arztes, die linke Datenbank D3 enthält zusätzliche medizinische Daten MD, wie z.B. in der Vergangenheit aufgenommene Bilddaten.

Zusätzlich zu der Abbildung der Indikationsdaten ID und ggf. weiterer Daten in die Ontologie O in Schritt I folgt eine Bereitstellung einer Steuerdaten-Bibliothek SB umfassend Steuerdatensätze SD, insbesondere in Form einer Workflow-Ontologie WO. Bei dieser Steuerdaten-Bibliothek SB kann es sich durchaus um eine eigenständige Datenbank handeln, deren Daten die Steuerdatensätze SD sind. Es wird im Folgenden aber einfach davon ausgegangen, dass die Steuerdaten-Bibliothek SB als eine Anzahl von Steuerdatensätzen SD in einer Datenbank vorliegt.

Es folgt in Schritt II eine automatische Ermittlung eines Steuerdatensatzes SD aus der Steuerdaten-Bibliothek SB in Abhängigkeit von den in die (Indikations-)Ontologie O abgebildeten Indikationsdaten ID. Dies geschieht mittels einer semantischen Inferenz und/oder numerischen Modellierung.

In Schritt II erfolgt dann eine Steuerung des medizintechnischen bildgebenden Systems 1 mit dem ermittelten Steuerdatensatz SD.

Figur 2 zeigt eine schematische Darstellung einer bevorzugten Steuereinrichtung 10 zur Steuerung eines medizintechnischen bildgebenden Systems 1, das hier - lediglich als ein Beispiel - ein Computertomographiesystem 1 ist. Bei der Steuereinrichtung 10 sind nur diejenigen Komponenten dargestellt, die für die Erläuterung der Erfindung wesentlich oder hilfreich sind, da der Aufbau und die Funktionsweise eines Computertomographiesystems 1 dem Fachmann bekannt sind und nicht genauer erläutert werden müssen.

Das Computertomographiesystem 1 weist in üblicher Weise einen Scanner 2 mit einer Gantry auf, in der eine Röntgenquelle 3 rotiert, die jeweils einen Patienten durchstrahlt, welcher mittels einer Liege 5 in einen Messraum der Gantry hineingeschoben wird, so dass die Strahlung auf einen der Röntgenquelle 3 jeweils gegenüberliegenden Detektor 4 trifft.

Eine Kernkomponente der Steuereinrichtung 10 ist hier ein Prozessor 11, auf dem verschiedene Komponenten in Form von Softwaremodulen realisiert sind. Die Steuereinrichtung 10 weist weiterhin eine Terminalschnittstelle 14 auf, an die ein Terminal 20 angeschlossen ist, über das ein Bediener die Steuereinrichtung 10 und somit das Computertomographiesystem 1 bedienen kann. Eine weitere Schnittstelle 15 ist eine Netzwerkschnittstelle zum Anschluss an einen Datenbus 21, um so eine Verbindung zu einem RIS bzw. PACS herzustellen. Über diesen Datenbus 21 werden in diesem Falle auch die Indikationsdaten ID und ggf. weitere Daten des Patientendatensatzes PD oder anderer Datenbanken bereitgestellt.

Über eine Steuerschnittstelle 13 kann von der Steuereinrichtung 10 der Scanner 2 angesteuert werden, d.h. es werden z.B. die Rotationsgeschwindigkeit der Gantry, die Verschiebung der Liege 5 und die Röntgenquelle 3 selbst gesteuert. Über eine Akquisitionsschnittstelle 12 werden die Rohdaten RD aus dem Detektor 4 ausgelesen. Weiterhin weist die Steuereinrichtung 10 eine Speichereinheit 16 auf. In dem hier gezeigten Fall umfasst die Speichereinheit 16 die komplette Steuerdaten-Bibliothek SB mit den einzelnen Steuerdatensätzen SD. Die Speichereinheit 16 ist über die Schnittstelle 17 mit dem Prozessor 11 verbunden. Theoretisch wäre es alternativ möglich, auch über ein Netzwerk oder den Datenbus 21 Zugriff auf eine Steuerdaten-Bibliothek SB zu erhalten. Die Steuerdaten-Bibliothek SB könnte auch direkt im Speicherbereich des Prozessors 11 implementiert sein.

Eine weitere Komponente auf dem Prozessor 11 ist eine Überführungseinheit 8, welche dazu ausgelegt ist, die Indikationsdaten ID und ggf. auch andere Daten in eine Ontologie O zu überführen.

Des Weiteren ist auf dem Prozessor 11 eine Ermittlungseinheit 9 vorhanden, welche zur Ermittlung eines Steuerdatensatzes SD aus der Steuerdaten-Bibliothek SB in Abhängigkeit von den in die Ontologie O abgebildeten Indikationsdaten ID, mittels einer semantischen Inferenz und/oder numerischen Modellierung, ausgelegt ist.

Eine weitere Komponente auf dem Prozessor 11 ist eine Bilddaten-Rekonstruktionseinheit 18, mit welcher aus den über die Datenakquisitions-Schnittstelle 12 erhaltenen Rohdaten RD die gewünschten Bilddaten rekonstruiert werden. Diese können dann der nachfolgend beschriebenen bevorzugten Ausführungsform zur Verfügung stehen.

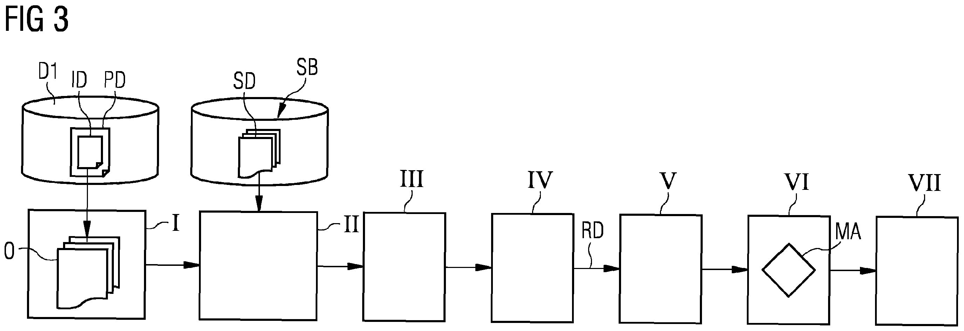

Figur 3 zeigt ein Blockdiagramm zur Verdeutlichung des Ablaufs einer bevorzugten Ausführungsform des erfindungsgemäßen Verfahrens. Wie in Figur 1 werden Indikationsdaten ID und ggf. weitere Patientendaten PD oder die im Rahmen der Erläuterungen zu Figur 1 genannten Daten in einem Schritt I in einer Ontologie O abgebildet.

In einem nachfolgenden Schritt II wird wie ebenfalls in Figur 1 beschrieben ein Steuerdatensatz SD aus einer Steuerdaten-Bibliothek SB ermittelt.

In Schritt III erfolgt eine Steuerung eines medizintechnischen bildgebenden Systems 1, was in Schritt IV in der Aufnahme von Rohdaten RD eines Bildes resultiert.

Diese Rohdaten RD werden dann an eine Bildrekonstruktionseinheit 18 übergeben, die in Schritt V eine Rekonstruktion der Rohdaten RD entsprechend der Vorgaben des Steuerdatensatzes SD vornimmt. Es ergeben sich daraus Bilddaten, die ein Befunder einsehen könnte.

In Schritt VI werden diese Bilddaten von einem Messdatendarstellungs-Algorithmus MA, der basierend auf den Indikationsdaten ID ausgewählt wird, bearbeitet und eine Präsentation erstellt, die ggf. auch noch weitere Daten enthalten kann. Idealer Weise werden die Bilder entsprechend der Indikationsdaten ID so dargestellt, wie es zur optimalen Befundung für das spezifische Krankheitsbild am besten ist.

In einem letzten Schritt VII werden die solcherart verarbeiteten Bilder und ggf. zusätzlichen Daten für einen Befunder ausgegeben, z.B. ausgedruckt und/oder an einem Bildschirm dargestellt.

Es wird abschließend noch einmal darauf hingewiesen, dass es sich bei den vorhergehend detailliert beschriebenen Verfahren sowie bei der dargestellten Steuereinrichtung 10 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriff "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Steuerung eines medizintechnischen bildgebenden Systems (1), umfassend die Schritte:
- Bereitstellung eines Datensatzes (PD) eines Patienten (P) enthaltend Indikationsdaten (ID),
- Abbildung zumindest der Indikationsdaten (ID) in eine Ontologie (O),
- Bereitstellung einer Steuerdaten-Bibliothek (SB) umfassend Steuerdatensätze (SD) für zumindest ein medizintechnisches bildgebendes System (1),
- automatische Ermittlung eines Steuerdatensatzes (SD) aus der Steuerdaten-Bibliothek (SB) in Abhängigkeit von den in die Ontologie (O) abgebildeten Indikationsdaten (ID), mittels einer Inferenz und/oder numerischen Modellierung auf Basis einer voreingestellten Zuordnung von semantischen Objekten innerhalb der Ontologie,
- Steuerung des medizintechnischen bildgebenden Systems (1) mit dem ermittelten Steuerdatensatz (SD),
- Rekonstruieren und/oder grafisches Nachbearbeiten eines Bilddatensatzes, wobei das Rekonstruieren und/ oder grafische Nachbearbeiten eines Bilddatensatzes automatisch oder halbautomatisch erfolgt basierend auf dem ermittelten Steuerdatensatz nach der Steuerung des ausgewählten medizintechnischen bildgebenden Systems und einer Aufnahme des Bilddatensatzes durch das medizintechnische bildgebende System.

2. Verfahren nach Anspruch 1, wobei die Steuerdaten-Bibliothek (SB) Steuerdatensätze (SD) für zumindest zwei bildgebende medizintechnisches Systeme (1) umfasst, wobei vor der automatischen Ermittlung eines Steuerdatensatzes (SD) eine Auswahl eines medizintechnischen bildgebenden Systems (1) erfolgt, mit dem eine Aufnahme von Bereichen des Körpers des Patienten (P) durchgeführt werden soll.

3. Verfahren nach einem der vorangehenden Ansprüche, wobei zusätzlich zu den Indikationsdaten (ID) weitere Daten des Patienten (PD, PD2) auf die Ontologie abgebildet werden, bevorzugt
- weitere Daten im Datensatz des Patienten (P), insbesondere Daten der Gruppe Alter, Gewicht, Größe, ethnische Zugehörigkeit, Geschlecht, und/oder
- weitere Daten einer Patientengruppe,
wobei die weiteren Daten des Patienten (PD, PD2) insbesondere aus einem Krankenhausinformationssystem und/oder einem Radiologieinformationssystem abgerufen werden.

4. Verfahren nach einem der vorangehenden Ansprüche, wobei die Ontologie zusätzliche medizinische Daten (MD) der Gruppe medizinische Vorgeschichte, Vorerkrankungen, allgemeiner Gesundheitszustand, Scanprotokolle, Kontrastprotokolle und weitere Daten eines lexikalischen Werks enthält.

5. Verfahren nach einem der vorangehenden Ansprüche, wobei die Steuerdaten-Bibliothek (SB) Daten im RadLex Playbook-Standard umfasst,
wobei diese Daten bevorzugt auf einem vorangegangenen Scanprotokoll und/oder aktuellen oder vorangegangen Untersuchungen des Patienten oder einer Patientengruppe basieren.

6. Verfahren nach einem der vorangehenden Ansprüche, wobei zur Ermittlung des Steuerdatensatzes (SD) eine modellierende Workflow-Ontologie (WO) verwendet wird, wobei die Workflow-Ontologie (WO) Schritte der Scanprozedur, benötigte Eingangs- und Ausgangsinformationen und verfügbare Steuerdaten, welche die gesamte Scan-Knowledge beinhalten, modelliert, und
wobei die Workflow-Ontologie (WO) bevorzugt ständig mit Informationen über medizinische Erfordernisse oder neue Steuerdaten erweitert oder aktualisiert wird.

7. Verfahren nach einem der vorangehenden Ansprüche, wobei die Inferenz und/oder numerischen Modellierung
- ausnutzt, dass die logischen Zusammenhänge der Daten des Datensatzes (PD) des Patienten (P) und der Steuerdaten-Bibliothek (SB) zu den Indikationsdaten (ID) passen müssen und nur diejenigen Daten zur Ermittlung hinzugezogen werden, welche zu den Indikationsdaten (ID) passen, und/oder
- numerische Informationen des Patienten (P) oder der Patientengruppe ausnutzt, bevorzugt mittels maschineller Lernprozeduren.

8. Verfahren nach einem der vorangehenden Ansprüche, wobei nach der Steuerung des ausgewählten medizintechnischen bildgebenden Systems (1) und einer Aufnahme eines Bilddatensatzes (RD) durch das medizintechnische bildgebende System (1) der Bilddatensatz (RD) automatisch oder halbautomatisch rekonstruiert wird und/oder grafisch nachbearbeitet wird,
wobei zur Bildrekonstruktion eine modellierende Workflow-Ontologie verwendet wird, welche bevorzugt ständig mit Informationen über Rekonstruktionsprotokolle erweitert oder aktualisiert wird.

9. Verfahren nach einem der vorangehenden Ansprüche, wobei nach der Steuerung des ausgewählten medizintechnischen bildgebenden Systems (1) und einer Aufnahme eines Bilddatensatzes (RD) durch das medizintechnische bildgebende System (1) der Bilddatensatz (RD) durch einen Messdatendarstellungs-Algorithmus (MA) automatisch für die Anzeige an einen Befunder aufbereitet wird, wobei der Messdatendarstellungs-Algorithmus (MA) basierend auf den Indikationsdaten (ID) und/oder dem Steuerdatensatz (SD) automatisch aus einer Darstellungsbibliothek ermittelt wird.

10. Verfahren nach Anspruch 9, wobei dem Steuerdatensatz (SD) zusätzlich während oder nach seiner Ermittlung weitere Daten (PD, PD2, MD) zugeordnet werden, insbesondere basierend auf der Ontologie (O) enthaltend Daten des Patienten (P), einer Patientengruppe und/oder eines lexikalischen Werks, wobei diese weiteren Daten (PD, PD2, MD) dem Messdatendarstellungs-Algorithmus (MA) Informationen zur Darstellung der Messdaten liefern.

11. Verfahren nach einem der Ansprüche 9 oder 10, wobei mittels des Messdatendarstellungs-Algorithmus (MA) zusätzlich zu dem Bilddatensatz (RD) auch Daten des Datensatzes (PD) des Patienten (P) und vorzugsweise ebenfalls Daten einer lexikalischen Datenbank, automatisch für die Anzeige an einen Befunder aufbereitet werden.

12. Steuereinrichtung (10) zur Steuerung eines medizintechnischen bildgebenden Systems (1), nach einem Verfahren gemäß einem der vorangehenden Ansprüche, umfassend
- eine Schnittstelle (15) zur Bereitstellung eines Datensatzes (PD) eines Patenten (P) enthaltend Indikationsdaten (ID),
- eine Schnittstelle (17) und/oder eine Datenbank (16) zur Bereitstellung einer Steuerdaten-Bibliothek (SB) umfassend Steuerdatensätze (SD) für zumindest ein medizintechnisches bildgebendes System (1),
- eine Überführungseinheit (8) ausgelegt zur Überführung zumindest der Indikationsdaten (ID) in eine Ontologie (O),
- eine Ermittlungseinheit (9), welche zur Ermittlung eines Steuerdatensatzes (SD) aus der Steuerdaten-Bibliothek (SB) in Abhängigkeit von den in die Ontologie (O) abgebildeten Indikationsdaten (ID), mittels einer semantischen Inferenz und/oder numerischen Modellierung auf Basis einer voreingestellten Zuordnung von semantischen Objekten innerhalb der Ontologie, ausgelegt ist,
- wobei die Steuereinrichtung ausgelegt ist für Rekonstruieren und/oder grafisches Nachbearbeiten eines Bilddatensatzes, wobei das Rekonstruieren und/oder grafische Nachbearbeiten automatisch oder halbautomatisch erfolgt basierend auf dem ermittelten Steuerdatensatz nach der Steuerung des ausgewählten medizintechnischen bildgebenden Systems und einer Aufnahme des Bilddatensatzes durch das medizintechnische bildgebende System.

13. Medizintechnisches bildgebendes System (1), welches eine Steuereinrichtung (10) nach Anspruch 12 umfasst.

14. Computerprogrammprodukt mit einem Computerprogramm, welches direkt in eine Speichereinrichtung einer Steuereinrichtung (10) eines medizintechnischen bildgebenden Systems (1), ladbar ist, mit Programmabschnitten, um alle Schritte des Verfahrens nach einem der Ansprüche 1 bis 11 auszuführen, wenn das Computerprogramm in der Steuereinrichtung (1) ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 11 auszuführen, wenn die Programmabschnitte von der Rechnereinheit ausgeführt werden.

## Claims

1. Method for controlling a medical imaging system (1), comprising the steps:
- Providing a data record (PD) for a patient (P), containing indication data (ID),
- Mapping at least the indication data (ID) into an ontology (O),
- Providing a control data library (SB) comprising control data records (SD) for at least one medical imaging system (1) ,
- Automatically determining a control data record (SD) from the control data library (SB) as a function of the indication data (ID) mapped into the ontology (O), by means of an inference and/or numerical modelling on the basis of a preset allocation of semantic objects within the ontology,
- Controlling the medical imaging system (1) with the determined control data record (SD),
- Reconstructing and/or graphically post-processing an image data record, wherein the reconstructing and/or graphical post-processing takes place automatically or semi-automatically on the basis of the determined control data record after the controlling of the selected medical imaging system and a recording of the image data record by the medical imaging system.

2. Method according to claim 1, wherein the control data library (SB) comprises control data records (SD) for at least two medical imaging systems (1), wherein before the automatic determination of a control data record (SD) a selection of a medical imaging system (1) is effected, with which a recording of regions of the body of the patient (P) should be performed.

3. Method according to one of the preceding claims, wherein in addition to the indication data (ID) further data of the patient (PD, PD2) is mapped onto the ontology, preferably
- Further data in the data record of the patient (P), in particular data of the group consisting of age, weight, height, ethnicity, gender, and/or
- Further data of a patient group,
wherein the further data of the patient (PD, PD2) is in particular retrieved from a hospital information system and/or a radiology information system.

4. Method according to one of the preceding claims, wherein the ontology contains additional medical data (MD) of the group consisting of previous medical history, pre-existing conditions, general state of health, scan protocols, contrast protocols and further data from a lexical corpus.

5. Method according to one of the preceding claims, wherein the control data library (SB) comprises data in the RadLex Playbook standard,
wherein this data is preferably based on a previous scan protocol and/or current or previous examinations of the patient or a patient group.

6. Method according to one of the preceding claims, wherein for determining the control data record (SD) a modelling workflow ontology (WO) is used, wherein the workflow ontology (WO) models steps of the scan procedure, necessary input and output information and available control data, which contains the totality of scan knowledge, and
wherein the workflow ontology (WO) is preferably constantly expanded or updated with information regarding medical requirements or new control data.

7. Method according to one of the preceding claims, wherein the inference and/or numerical modelling
- makes use of the fact that the logical relationships of the data of the data record (PD) of the patient (P) and the control data library (SB) has to match the indication data (ID), and only the data which matches the indication data (ID) is called upon for the determination, and/or
- makes use of numerical information of the patient (P) or the patient group, preferably by means of machine learning procedures.

8. Method according to one of the preceding claims, wherein after the controlling of the selected medical imaging system (1) and a recording of an image data record (RD) by the medical imaging system (1), the image data record (RD) is automatically or semi-automatically reconstructed and/or graphically post-processed,
wherein a modelling workflow ontology is preferably used for the image reconstruction, which is particularly preferably constantly expanded or updated with information regarding reconstruction protocols.

9. Method according to one of the preceding claims, wherein after the controlling of the selected medical imaging system (1) and a recording of an image data record (RD) by the medical imaging system (1), said image data record (RD) is automatically prepared for display to a reporting physician by a measurement data representation algorithm (MA), wherein the measurement data representation algorithm (MA) is determined from a representation library automatically on the basis of the indication data (ID) and/or the control data record (SD).

10. Method according to claim 9, wherein further data (PD, PD2, MD) is additionally allocated to the control data record (SD) during or after its determination, in particular based on the ontology (O) containing data of the patient (P), a patient group and/or a lexical corpus, wherein this further data (PD, PD2, MD) supplies the measurement data representation algorithm (MA) with information for representing the measurement data.

11. Method according to one of claims 9 or 10, wherein, in addition to the image data record (RD), data of the data record (PD) of the patient (P) and preferably likewise data of a lexical database, are automatically also prepared by means of the measurement data representation algorithm (MA) for display to a reporting physician.

12. Control facility (10) for controlling a medical imaging system (1), according to a method according to one of the preceding claims, comprising
- an interface (15) for providing a data record (PD) of a patient (P), containing indication data (ID),
- an interface (17) and/or a database (16) for providing a control data library (SB) comprising control data records (SD) for at least one medical imaging system (1),
- a transfer unit (8) configured for transferring at least the indication data (ID) into an ontology (O),
- a determination unit (9), which is configured for determining a control data record (SD) from the control data library (SB) as a function of the indication data (ID) mapped into the ontology (O), by means of a semantic inference and/or numerical modelling on the basis of a preset allocation of semantic objects within the ontology,
- wherein the control facility is configured for reconstructing and/or graphically post-processing an image data record, wherein the reconstructing and/or graphical post-processing takes place automatically or semi-automatically on the basis of the determined control data record after the controlling of the selected medical imaging system and a recording of the image data record by the medical imaging system.

13. Medical imaging system (1), which comprises a control facility (10) according to claim 12.

14. Computer program product with a computer program which can be loaded directly into a memory store of a control facility (10) of a medical imaging system (1), having program portions in order to carry out all the steps of the method according to one of claims 1 to 11 when the computer program is executed in the control device (1).

15. Computer-readable medium on which program portions that can be read in and executed by a computer unit are stored, in order to carry out all the steps of the method according to one of claims 1 to 11 when the program portions are executed by the computer unit.

## Revendications

1. Procédé de commande d'un système (1) d'imagerie de la technique médicale, comprenant les stades :
- on se procure un ensemble (PD) de données d'un patient (P) contenant des données (ID) d'indication,
- on reproduit au moins les données (ID) d'indication dans une ontologie (O),
- on se procure une bibliothèque (SB) de données de commande comprenant des ensembles (SD) de données de commande d'au moins un système (1) d'imagerie de la technique médicale,
- on détermine automatiquement un ensemble (SD) de données de commande, à partir de la bibliothèque (SB) de données de commande, en fonction des données (ID) d'indication reproduites dans l'ontologie (O), au moyen d'une inférence et/ou d'une modélisation numérique sur la base d'une affectation établie à l'avance d'objets sémantiques dans l'ontologie,
- on commande le système (1) d'imagerie de la technique médicale par l'ensemble (SD) de données de commande déterminé,
- on reconstruit et/ou on retraite graphiquement un ensemble de données d'image, la reconstruction et/ou le retraitement graphique d'un ensemble de données d'image s'effectuant automatiquement ou semi-automatiquement sur la base de l'ensemble de données de commande déterminé, après la commande du système d'imagerie de la technique médicale sélectionné et un enregistrement de l'ensemble de données d'image par le système d'imagerie de la technique médicale.

2. Procédé suivant la revendication 1, dans lequel la bibliothèque (SB) de données de commande comprend des ensembles (SD) de données de commande d'au moins deux systèmes (1) de la technique médicale d'imagerie, dans lequel, avant la détermination automatique d'un ensemble (SD) de données de commande, il s'effectue une sélection d'un système (1) d'imagerie de la technique médicale, par lequel un enregistrement de parties du corps du patient (P) doit être effectué.

3. Procédé suivant l'une des revendications précédentes, dans lequel on reproduit sur l'ontologie, supplémentairement aux données (ID) d'indication, d'autres données (PD, PD2) du patient, de préférence
- d'autres données dans l'ensemble de données du patient (P), en particulier des données du groupe âge, poids, taille, appartenance ethnique, sexe, et/ou
- d'autres données d'un groupe de patients,
dans lequel on appelle les autres données du patient (PD, PD2), en particulier dans un système d'information d'hôpital ou dans un système d'information de radiologie.

4. Procédé suivant l'une des revendications précédentes, dans lequel l'ontologie contient des données (MD) médicales supplémentaires, du groupe antécédents médicaux, maladies antérieures, état de santé général, protocole de scan, protocole de contraste et d'autres données d'un ouvrage lexical.

5. Procédé suivant l'une des revendications précédentes, dans lequel la bibliothèque (SB) de données de commande comprend des données dans RadLex playbook - standard,
dans lequel ces données reposent, de préférence, sur un protocole de scan précédent et/ou sur des examens en cours ou précédents du patient ou d'un groupe de patients.

6. Procédé suivant l'une des revendications précédentes, dans lequel, pour la détermination des ensembles (SD) de données de commande, on utilise une ontologie workflow (WO) modélisante, dans lequel l'ontologie workflow (WO) modélise des stades de la procédure scan, des informations nécessaires d'entrée et de sortie et des données de commande disponibles, qui contiennent la connaissance de scan d'ensemble, et dans lequel l'ontologie workflow (WO) est étendue ou mise à jour, de préférence en permanence par des informations sur des nécessités médicales ou des données de commande nouvelles.

7. Procédé suivant l'une des revendications précédentes, dans lequel l'inférence et/ou la modélisation numérique,
- exploite le fait que les relations logiques des données de l'ensemble (PD) de données du patient (P) et de la bibliothèque (SB) de données de commande doivent s'adapter aux données (ID) d'indication et ne doivent être mises à profit que les données de détermination, qui s'adaptent aux données (ID) d'indication, et/ou
- exploite des informations numériques du patient (P) ou du groupe de patients, de préférence au moyen de procédures d'apprentissage par machine.

8. Procédé suivant l'une des revendications précédentes, dans lequel, après la commande du système (1) d'imagerie de la technique médicale sélectionné et un enregistrement d'un ensemble (RD) de données d'image par le système (1) d'imagerie de la technique médicale, on reconstruit automatiquement ou semi-automatiquement et/ou on retraite graphiquement l'ensemble (RD) de données d'image, dans lequel, pour la reconstruction d'image, on utilise une workflow - ontologie modélisante, que l'on met à jour, de préférence en permanence, par des informations sur des protocoles de reconstruction.

9. Procédé suivant l'une des revendications précédentes, dans lequel, après la commande du système (1) d'imagerie de la technique médicale sélectionné et un enregistrement d'un ensemble (RD) de données d'image par le système (1) d'imagerie de la technique médicale, on traite automatiquement, pour l'affichage sur un diagnostiqueur, l'ensemble (RD) de données d'image par un algorithme (MA) de représentation de données de mesure, dans lequel on détermine l'algorithme (MA) de représentation de données de mesure sur la base des données (ID) d'indication et/ou de l'ensemble (SD) de données de commande automatiquement à partir d'une bibliothèque de représentation.

10. Procédé suivant la revendication 9, dans lequel on affecte d'autres données (PD, PD2, MD) à l'ensemble (SD) de données de commande supplémentairement pendant ou après sa détermination, en particulier sur la base de l'ontologie (O) contenant des données du patient (P), d'un groupe de patients et/ou d'un ouvrage lexical, dans lequel ces autres données (PD, PD2, MD) fournissent à l'algorithme (MA) de représentation de données de mesure des informations pour la représentation des données de mesure.

11. Procédé suivant l'une des revendications 9 ou 10, dans lequel, au moyen de l'algorithme (MA) de représentation de données de mesure, on traite automatiquement pour l'affichage sur un diagnostiqueur, en plus de l'ensemble (RD) de données d'image, également des données de l'ensemble (PD) de données du patient (P) et, de préférence également des données d'une base de données lexicale.

12. Dispositif (10) de commande pour la commande d'un système (1) d'imagerie de la technique médicale suivant un procédé suivant l'une des revendications précédentes, comprenant
- une interface (15) pour la mise à disposition d'un ensemble (PD) de données d'un patient (P) contenant des données (ID) d'indication,
- une interface (17) et/ou une base (16) de données pour la mise à disposition d'une bibliothèque (SB) de données de commande comprenant des ensembles (SD) de données de commande d'au moins un système (1) d'imagerie de la technique médicale,
- une unité (8) de transfert conçue pour le transfert d'au moins les indications (ID) d'indication dans une ontologie (O),
- une unité (9) de détermination, qui est conçue pour la détermination d'un ensemble (SD) de données de commande, à partir de la bibliothèque (SB) de données de commande, en fonction des données (ID) d'indication reproduites dans l'ontologie (O) au moyen d'une inférence sémantique et/ou d'une modélisation numérique sur la base d'une affectation établie au préalable d'objets sémantiques dans l'ontologie,
- dans lequel le dispositif de commande est conçu pour la reconstruction et/ou le retraitement graphique de l'ensemble de données d'image, la reconstruction et le retraitement graphique s'effectuant automatiquement ou semi-automatiquement sur la base de l'ensemble de données de commande déterminé, après la commande du système d'imagerie de la technique médicale sélectionné et un enregistrement de l'ensemble de données d'image par le système d'imagerie de la technique médicale.

13. Système (1) d'imagerie de la technique médicale, qui comprend un dispositif (10) de commande suivant la revendication 12.

14. Produit de programme d'ordinateur comprenant un programme d'ordinateur, qui peut être chargé directement dans un dispositif de mémoire d'un dispositif (10) de commande d'un système (1) d'imagerie de la technique médicale, comprenant des parties de programme pour exécuter tous les stades du procédé suivant l'une des revendications 1 à 11, lorsque le programme d'ordinateur est exécuté dans le dispositif (1) de commande.

15. Support, déchiffrable par ordinateur, sur lequel sont mises en mémoire des parties de programme pouvant être déchiffrées et exécutées par une unité informatique, afin d'exécuter tous les stades du procédé suivant l'une des revendications 1 à 11, lorsque les parties de programme sont exécutées par l'unité informatique.
